# EUROPEAN PATENT APPLICATION

(11) **EP 1 121 950 A1**
(43) Date of publication of application: **08.08.2001**
(21) Application number: 99970030.5
(22) Date of filing: 01.10.1999
(51) Int. Cl.: A61M 5/32, A61M 5/34

(54) **DRUG INJECTOR**

(30) Priority: 06.10.1998 JP 28390998
(71) Applicant: Nissho Corporation, Osaka-shi, Osaka 531-0073 (JP); Sunstar Inc., Takatsuki-shi, Osaka 569-1134 (JP)
(72) Inventor: HIRAYAMA, Toshikazu, Osaka-shi, Osaka 531-0073 (JP); HASEGAWA, Shuji, Osaka-shi, Osaka 531-0073 (JP); TOCHIYAMA, Toshio, Osaka-shi, Osaka 531-0073 (JP); EGUCHI, Tooru, Takatsuki-shi, Osaka 569-1044 (JP); SHIMIZU, Yasumitsu, Kusatsu-shi, Shiga 525-0023 (JP); YONEDA, Junko, Ibaraki-shi, Osaka 567-0009 (JP)
(74) Representative: Lemoine, Robert
(86) International application number: JP9905430
(87) International publication number: WO0020060

(57) **Abstract**

A drug syringe which is capable of applying a nozzle of an appropriate size and angle corresponding to a patient, and can prevent infection and make economical use of drugs by replacing the nozzle is disclosed. The drug syringe comprises a barrel 1 which is provided with a nozzle mounting portion 11 at a distal end thereof; a plunger 2 which is provided with a gasket 21 at a distal end thereof; and a nozzle 3 which is freely detachable from the nozzle mounting portion 11, wherein the nozzle 3 includes a mounting portion 31 to the barrel 1, and a discharging portion 32 which extends bending at a predetermined angle from the mounting portion 31.

## Description

### Field of the Invention

The present invention relates to a drug syringe, and more particularly, to a drug syringe for a local administration in medical field and the like, the tip of which is detachable and does not fall off even under high pressure.

### Background of the Invention

As a local administration syringe utilized in medical field, conventionally, cartridge-mounted and injector-type syringe have been known. The cartridge-mounted syringe is most often used when administering a local anesthetic drug, primarily, in the field of dentistry. The injector-type syringe is used as a disposable one which gives serious consideration to the aspect of hygiene, and is utilized as a bioadhesive filled container at a surgical operation in the medical field, and a drug-filled container for treating periodontal disease is known in dental field. With regard to a periodontal disease remedy, syringes for injecting a drug into a periodontal pocket to remove periodontpathic bacteria that propagate inside the periodontal pocket have been proposed in Japanese Patent Application Laid open No. 4-117959 or Japanese Patent Utility Model Registration No.3035448. The syringe disclosed in Japanese Patent Application Laid-open No. 4-117959 is a syringe on which a mark for decision is disposed at a position on the nozzle separated from the distal end position by a distance corresponding to the depth of a periodontal pocket, which constitutes the criteria for determining whether or not a drug is injected. The nozzle is disposed by being inclined at a predetermined angle relative to the axis at the distal end portion of the syringe body, and when used, the syringe nozzle is inserted into a periodontal pocket, and a determination to inject a drug or not is made by observing an appearance of the mark from the periodontal pocket and the disappearance and when injection is required, a plunger is depressed in the as-is state, and the drug can be injected from the nozzle.

However, although the regions to which a syringe is applied are numerous and varied, it was not possible to change the size of the nozzle in accordance with the state of the injection region (depth, shape etc. of the pocket) with the syringe disclosed in the above-mentioned Japanese Patent Application Laid-open No. 4-117959in wherein the nozzle and syringe body are integrally formed. Further, the drug loaded completely inside a syringe is seldom used up in one usage and the syringe must be discarded from the standpoint of preventing infections such as the AIDS and hepatitis in recent years even though ample drug remains, so that there was the drawback that it is uneconomical.

Accordingly, as a syringe in order to resolve the drawbacks of the above-mentioned drug syringe, a syringe which is provided with a nozzle mounting portion inclined at a predetermined angle on the top of a syringe body, and is capable of mounting a plurality of nozzles of different diameters and/or lengths is proposed (Japanese Utility Model Registration No.3035448). However, this syringe engages a nozzle in a tapered condition to a nozzle mounting portion which is formed in a tapered shape, and there is a danger that the nozzle falls off from the nozzle mounting portion during use when a substance of a high viscosity is injected. Further, because the inclination angle of the nozzle to the syringe body is established, it is actually impossible to change the angle without replacing the syringe main body when it is desirable to change the angle in accordance with the injection region.

### SUMMARY OF THE INVENTION

With the foregoing in view, it is an object of the present invention to provide a drug syringe which is capable of using a nozzle of an appropriate size and angle in accordance with an injection region, and which can prevent infection and make economical use of drugs by replacing a nozzle.

The present inventors, as a result of zealous studies to solve the above-mentioned problems, arrived at the idea of using a nozzle, a distal end of which is inclined at a predetermined angle relative to a proximal end, and accomplished the invention. That is, the present invention is a drug syringe comprising a barrel which is provided with a nozzle mounting portion at a distal end thereof; a plunger which is provided with a gasket capable of hermetically sliding along the inner wall of said barrel at a distal end thereof and is inserted from a proximal end of said barrel; and a nozzle which is freely detachable from said nozzle mounting portion, wherein said nozzle includes a mounting portion of the proximal end provided with means for mounting to said nozzle mounting portion and a discharging portion which extends bending at a predetermined angle from this mounting portion.

It is desirable that the mounting portions of the nozzle and the nozzle mounting portion of the barrel are formed so as to enable a luer lock. More specifically, the nozzle mounting portion is constituted with a distal end tip that engages with the inner cavity of the nozzle and female threads that are disposed concentrically on the outside of the distal end tip, and these female threads screw together with male threads disposed on the proximal end of the nozzle. The female threads can be integrally formed with the barrel, or can be disposed in a freely rotating condition on the outer wall of the barrel. Furthermore, the nozzle which includes a proximal end side mounting portion provided with means for mounting to the tip of the barrel and a discharging portion which extends bending at a predetermined angle from this mounting portion, can also be applied to an ordinary injector.

### BRIEF DESCRIPTION OF THE DRAWINGS

Fig. 1 is a longitudinal cross-sectional view showing one embodiment of the present invention; and
Fig. 2 is a longitudinal cross-sectional view showing another embodiment of the present invention.

### DESCRIPTION OF THE PREFERRED EMBODIMENTS

Next, the embodiments of the present invention will be explained by referring to the figures.

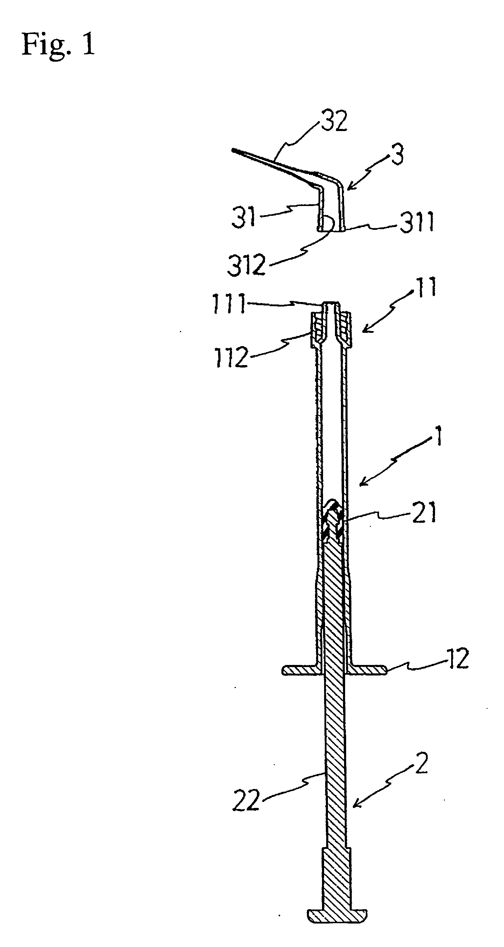

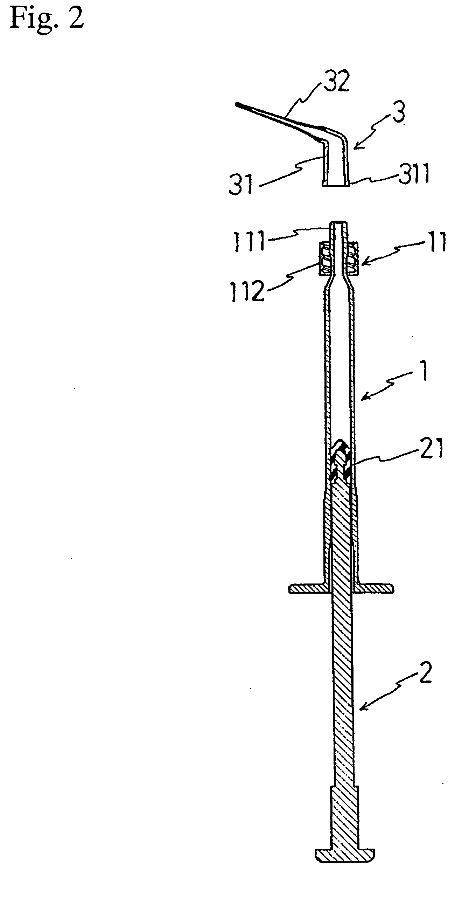

Fig. 1 is a longitudinal cross-sectional view showing one embodiment of the present invention, and Fig. 2 is a longitudinal cross-sectional view showing another embodiment.

A drug syringe of the present invention comprises a barrel 1 which is provided with a nozzle mounting portion 11 at a distal end thereof; a plunger 2 which is provided with a gasket 21 at a distal end thereof; and a nozzle 3 which is freely detachable with the nozzle mounting portion 11, wherein the nozzle 3 includes a mounting portion 31 for mounting to the barrel 1 and a discharging portion 32 which extends bending at a predetermined angle from this mounting portion 31.

The barrel 1 is a tubular container which is formed from a synthetic resin such as polypropylene, polyethylene, or cyclic polyolefin and has a flange 12 at the proximal end to be pushed by finger at an injection operation and is provided with the nozzle mounting portion 11 for mounting a nozzle 3 at the distal end. The nozzle mounting portion 11 ordinarily comprises a distal end tip 111 which is mounted and engaged with an inner cavity 312 of the mounting portion 31 of the nozzle 3 and female threads 112 which screw together with the hereinbelow-described male threads 311 disposed at the proximal end of the nozzle 3. The female threads 112 are a hood-shaped member concentrically disposed on the outside of the distal end tip 111, and as shown in Fig. 1, can be integrally formed with a barrel 1, or, as shown in Fig. 2, can also be disposed in a freely turning condition on the outer wall of the barrel 1, and is constituted so as to make the orientation of a nozzle 3 changeable at will. Furthermore, the distal end tip 111 is ordinarily disposed so as to protrude toward the distal end side away from the female threads 112, as shown in Fig. 1 and Fig. 2.

The plunger 2 is a discharging member which is provided with a gasket 21 at the distal end of the plunger rod 22 and is inserted from the proximal end of the barrel 1. The plunger rod 22 is a rod-shaped member formed from a synthetic resin such as polypropylene, polyethylene, polycarbonate, polystyrene or ABS and has a head 221 at the distal end. The gasket 21 is a closing member typically formed from an elastic rubber material such as butyl rubber or isoprene rubber, is mounted to the head 221 of the plunger rod 22 and is capable of sliding hermetically along the inner wall of the barrel 2 which has been inserted.

The nozzle 3 is a hollow member formed from the same synthetic resin as the barrel 1 and includes a mounting portion 31 which is provided with means for mounting to the nozzle mounting portion 11 of the barrel 1. and a discharging portion 32 which extends bending at a predetermined angle from this mounting portion 31. Means for mounting to the nozzle mounting portion 11 is disposed at the proximal end of the mounting portion 31 and male threads 311 which are coupling means corresponding to the female threads 112 of the barrel 1 are ordinarily utilized. The male threads 311 are ordinarily disposed axisymmetrically in a double threaded condition (referred to as a double threaded screw), and are capable of being screwed together with the female threads 112 of the barrel 1. The discharging portion 32 is a part which extends toward the distal end by being bent at a predetermined angle from the mounting portion 31 and is formed so as to become thinner at the tip in a tapered shape. The size and bending angle of the discharging portion 32 are determined in line with the state of an injection region of a patient, and ordinarily the size of the discharging portion 32 is from 3 to 20 mm, and the bending angle is ordinarily from 0º to 90º.

Furthermore, the nozzle 3 can be molded entirely in one step, or in order to improve the dimensional precision of the discharging portion 32, either the whole nozzle can be formed by molding an insert after forming a discharging portion 32, or a mounting portion 31 and a discharging portion 32 can be formed separately, and thereafter bonded together (in this case, the mounting portion 31 must be bent at a predetermined angle at the distal end part thereof). Further, the drug syringe of the present invention can also be provided as a pre-filled syringe in which a drug is previously loaded into the inside of the barrel 1, and the nozzle mounting portion 11 at the distal end is closed with a cap (not shown in the figures).

As is clear from the above explanation, it is possible to select a nozzle of the appropriate size and bending angle in accordance with the state of the injection region of a patient at time of use by using the drug syringe of the present invention. Further, because the nozzle can be replaced at time of reuse, it is possible to prevent infection from the nozzle part.

## Claims

1. A drug syringe comprising:
a barrel which is provided with a nozzle mounting portion at a distal end thereof;
a plunger which is provided with a gasket capable of sliding hermetically along an inner wall of the barrel at a distal end thereof and inserted from a proximal end of said barrel; and
a nozzle which is freely detachable with said nozzle mounting portion,
wherein said nozzle includes a mounting portion on the proximal end side which is provided with means for mounting to said nozzle mounting portion and a discharging portion which extends bending at a predetermined angle from this mounting portion.

2. The drug syringe according to claim 1, wherein the mounting portion of the nozzle and the nozzle mounting portion of the barrel are formed so as to enable a luer lock.

3. The drug syringe according to claim 2, wherein the nozzle mounting portion comprises a distal end tip that engages with the inner cavity of the nozzle and female threads which are disposed concentrically on the outside of the distal end tip, and said female threads are constituted so as to screw together with male threads disposed on the proximal end of the nozzle.

4. The drug syringe according to claim 3, wherein the female threads are integrally formed with the barrel.

5. The drug syringe according to claim 3, wherein the female threads are disposed in a freely rotatable condition on the outer wall of the barrel.

6. A nozzle for a drug syringe comprising:
a mounting portion on the proximal end side which is provided with means for mounting to a tip of a barrel; and
a discharging portion which extends bending at a predetermined angle from this mounting portion.
